# EUROPEAN PATENT APPLICATION

(11) **EP 2 529 759 A1**
(43) Date of publication of application: **05.12.2012**
(21) Application number: 11004554.9
(22) Date of filing: 03.06.2011
(51) Int. Cl.: A61L 2/10, A61B 19/02, A61G 12/00, A61L 9/20, F24F 3/16

(54) **Apparatus and method for an inactivation of proteinogenic allergens**

(71) Applicant: Virobuster GmbH, 53578 Windhagen (DE)
(72) Inventor: Nanninga, Herman, 53578 Windhagen (DE); Gerstenmeier, Jürgen, 53578 Windhagen (DE)
(74) Representative: Koepe & Partner

(57) **Abstract**

The present invention relates to an apparatus (1) and a method for inactivating allergens in a fluid by UV radiation treatment, said apparatus (1) comprising

- a housing (10) comprising and extending between a fluid inlet (11) and a fluid outlet (12);
- a fan (20) for generating and stimulating a fluid flow from the fluid inlet (11) through the housing (10) to the fluid outlet (12);
- a UV treatment chamber (30) in said housing (10) downstream relative to said fluid inlet (11), the UV treatment chamber (30) comprising at least one UV radiation source (31) for exposing said fluid flow to UV radiation for inactivating allergenic molecules present in said fluid flow; and
- control means arranged in said fluid flow from the fluid inlet (11) through the housing (10) to the fluid outlet (12) so as to control parameters relevant for the allergen inactivation in said fluid.

## Description

The present invention relates to an apparatus for the inactivation of proteinogenic allergens. More specifically, the invention relates to an apparatus suitable for the inactivation of proteinogenic allergens in a fluid stream, particularly in an air stream, by UV radiation applied or irradiated to the fluid or air stream. The invention also relates to a method for the inactivation of proteinogenic allergens by UV radiation.

The number of people suffering from allergies and the allergies' physiological impacts is increasing. Since allergens potentially responsible for causing allergic reactions are ubiquitous, many efforts have been invested into reducing the volume-related amount of allergens in the environmental fluids, particularly in the environmental air. These efforts were, however, not overwhelmingly successful. One of the reasons may be that allergens, particularly proteinogenic allergens, are not only transported by the air entering into or flooding a room, for example a room where humans live, but also may enter into such a room by animals living in said room together with the human(s) or living in a room which is entered by humans repeatedly. Examples are proteinogenic allergens based on animal hair (cats, dogs, horses). Another reason may result from the fact that proteinogenic allergens may grow, and multiply, within a room where humans live; an example may be house dust and mites contained therein, which often cause allergic reactions in children, in the meantime. Finally, allergens including proteinogenic allergens cannot be completely excluded from entering a room, because the complete isolation of rooms from the outside environment is not taken into consideration due to the desire not to live in an artificially maintained air environment, for whatever reason.

Apparatus for inactivating microorganisms and allergens were already proposed in the prior art. The document US-A 2009/0,280,027 proposed an apparatus for treating air, with the aim of "killing" and/or mineralizing various microorganisms, including allergens, and oxidizing volatile organic components, using a photocatalytic reaction, wherein the apparatus comprises (i) at least one reactor bed comprising a plurality of media coated at least partly with a photocatalytically active substance and a plurality of UV light sources suitable to emit UV light at wavelengths within a range of 180 to 390 nm and located within and substantially surrounded by the plurality of photocatalyst-coated media so that said media may receive UV light from the UV light sources; and (ii) an air-handling unit in communication with the reactor bed and adapted to move untreated air into and through the reactor bed and into contact with at least a portion of the plurality of photocatalyst-coated media and subsequently remove treated air from the reactor bed. In said document, the process occurring in the reactor is described as a process of generating hydroxyl radicals by the photocatalyst substance coated on the media through the photons of the UV radiation, which hydroxyl radicals are inactivating and mineralizing microorganisms and are oxidizing volatile organic compounds.

The document WO 2005/039,659 describes an air treatment device comprising a housing comprising an air inlet and an air outlet; a fan for stimulating an airflow through the housing from the air inlet to the air outlet; a UV treatment chamber downstream relative to said air inlet, the UV treatment chamber comprising at least one UV radiation source for exposing said airflow to UV radiation for inactivating microorganisms present in said airflow. The treatment of air streams for removing allergens, particularly proteinogenic allergens, is not addressed in said document.

Hence, it was an object of the present invention to provide a method and an apparatus for inactivating allergens, particularly proteinogenic allergens, in fluid streams and particularly in air streams in a room effectively. It was a further object of the invention to provide a method and an apparatus for inactivating (proteinogenic) allergens in the air of a room where humans live without that such air is treated permanently. Moreover, it was an object of the present invention to provide a method and an apparatus for inactivating (proteinogenic) allergens emerging from allergen sources present in said room or even living with humans in said room.

It was surprisingly found by the present inventors that allergens, particularly proteinogenic allergens, can be inactivated effectively in a fluid, particularly in air, present in a room by using ultraviolet radiation of a specific wavelength as the inactivating medium.

Hence, the present invention relates to an apparatus for inactivating allergens in a fluid by UV radiation treatment, said apparatus comprising
- a housing comprising and extending between a fluid inlet and a fluid outlet;
- a fan for generating and stimulating a fluid flow from the fluid inlet through the housing to the fluid outlet;
- a UV treatment chamber in said housing downstream relative to said fluid inlet, the UV treatment chamber comprising at least one UV radiation source for exposing said fluid flow to UV radiation for inactivating allergenic molecules present in said fluid flow; and
- control means arranged in said fluid flow from the fluid inlet through the housing to the fluid outlet so as to control parameters relevant for the allergen inactivation in said fluid.

Preferred embodiments of the apparatus are claimed in the subclaims dependant upon claim 1.

The invention also relates to a method for inactivating allergens in a fluid by UV radiation treatment in an apparatus comprising
- a housing comprising and extending between a fluid inlet and a fluid outlet;
- a fan for stimulating a fluid flow from the fluid inlet through the housing to the fluid outlet;
- a UV treatment chamber in said housing preferably (but not necessarily) downstream relative to said fluid inlet, the UV treatment chamber comprising at least one UV radiation source for exposing said fluid flow to UV radiation for inactivating allergenic molecules present in said fluid flow; and
- control means arranged in said fluid flow from the fluid inlet through the housing to the fluid outlet so as to control parameters relevant for the allergen inactivation in said fluid,
- said method comprising at least one of the following steps:
- guiding at least a part of said fluid flow through at least a part of the housing;
- directing said fluid flow towards, and at least partially into, said UV treatment chamber;
- exposing said fluid flow in said UV treatment chamber to UV radiation for inactivating allergenic molecules present in said fluid flow; and
- controlling said exposure step, and optionally other steps in combination, with respect to at least one of the parameters relevant for the allergen inactivation in said fluid.

The present invention is now further explained in detail by means of its preferred embodiments, which are given for exemplary purposes in order to allow a better understanding of the invention, but should not be construed to restrict the invention to only these preferred embodiments.

The term "comprise", "comprises" or "comprising" as used in the present specification and claims, for example in claim 1 (apparatus claim), is defined to have the meaning that said apparatus of the invention may comprise (or include) (i) at least one component of the apparatus following said term "comprise" or "comprises" or "comprising" or may comprise (or include) (ii) two or more components of the apparatus following said term "comprise" or "comprises" or "comprising", or that (iii) further components (more specifically defined below) may also be comprised by the apparatus of the invention.

The term "comprise" "comprises" or "comprising" as used in the present specification and claims is, however, also defined for the present invention to optionally include cases where the apparatus of the invention mainly (or even exclusively) consists of (i) at least one component of the apparatus following said term "comprise" or "comprises" or "comprising" or mainly (or even exclusively) consists of (ii) two or more components of the apparatus following said term "comprise" or "comprises" or "comprising", optionally together with any necessary component a skilled person may optionally additionally include into such an apparatus in order to achieve the object of the invention, or may even include cases where the component of the apparatus following said term "comprise" or "comprises" or "comprising" exclusively consists of (i) at least one component named or exclusively consists of (ii) two or more components named, optionally together with any necessary component a skilled person may include into such an apparatus in order to achieve the object of the invention.

In other words: The term "comprise" or "comprises" or "comprising" may have, in the present specification and claims, the meanings of describing an exhaustive or, alternatively, a non-exhaustive enumeration of elements, without that, in the former case, embodiments are excluded which, for example in subclaims of the present application, claim further features, which are beneficial or advantageous but not essential for the present invention.

The present invention is further described by referring to Figures. These Figures show preferred embodiments of the invention and are given for allowing a better understanding of the invention. However, the invention is not restricted by these preferred embodiments. In the Figures,
Figure 1 shows a sketch of a side section view of the apparatus for inactivating allergens according to the invention; Figure 1 is not necessarily showing the relative dimensions of the apparatus of the present invention by scale, i. e. with the exact relative dimensions of length, diameter and arrangement of the parts of the apparatus; these are shown in Figure 1 in one exemplary embodiment only and actually may be different from those shown in the present Figure 1;

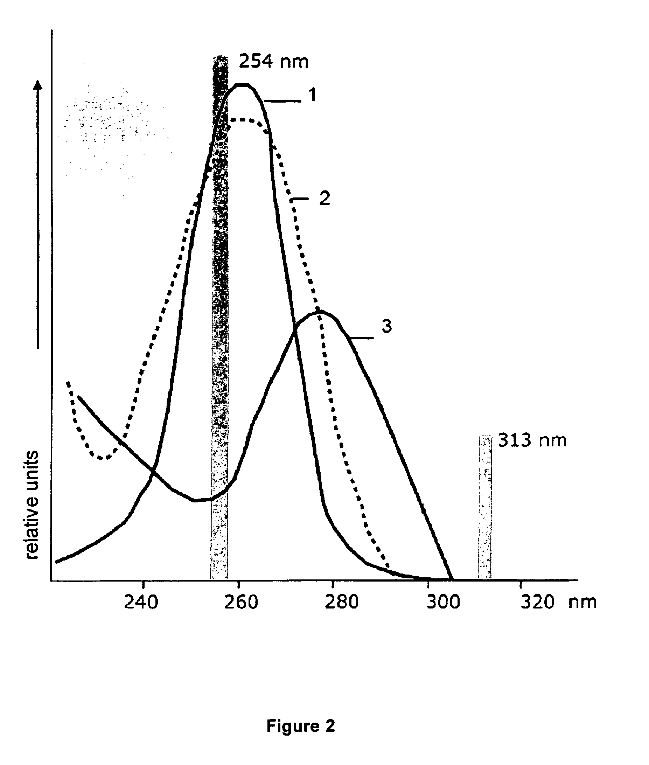

Figure 2 shows a plot of the spectral absorption graphs relevant for the present invention, wherein "1" is the graph showing the spectral maximum peak for microrganism cell inactivation; "2" is the graph showing the spectral maximum absorption of nucleic acids; and "3" is the graph showing the spectral maximum absorption of proteins; and
Figure 3 shows a plot of the effect of the inactivation of hazelnut protein Cor a1 by UV-C radiation according to the wavelength irradiated: An inactivation of said proteinogenic allergen at 278 nm is more than 60 % higher than that at 254 nm.
Figures 4 and 5 show graphs representing the results of an inactivation of the allergens Can f1 and Cor a1 by 278 nm UV-C radiation

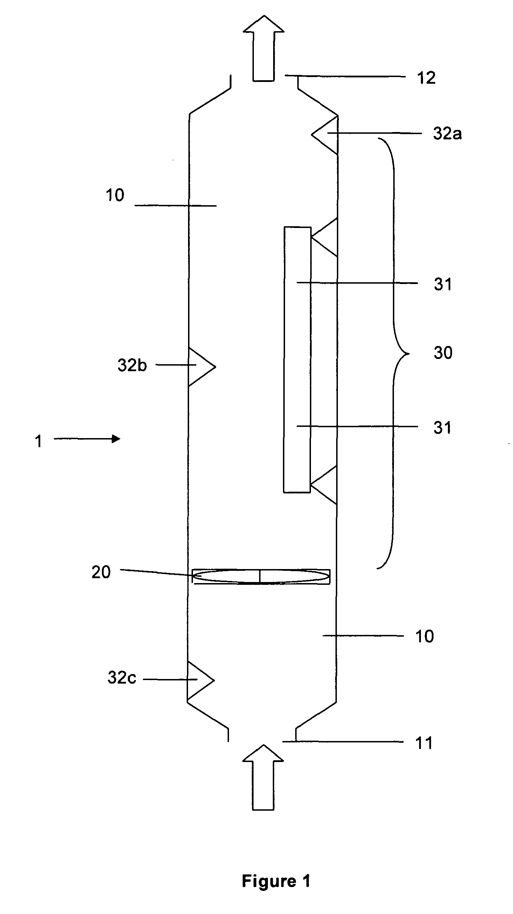

Reference is now made to Figure 1. Figure 1 shows one exemplary (but not restrictive) apparatus of the invention. In accordance with the invention, apparatus 1 is capable of being used for inactivating allergens by radiation, for example by UV radiation. In accordance with the invention, the apparatus 1 comprises a housing 10 comprising, and extending between, a fluid inlet 11 and a fluid outlet 12. The housing 10 may have any desired shape considered suitable by a skilled person for the purposes of the invention. In a preferred embodiment of the invention, the housing 11 has a cylindrical shape, even more preferably with an axis along the flow direction of a fluid flow, which axis, in an even more preferred embodiment, may be arranged in a vertical direction. In a particularly preferred embodiment, the cylindrical housing 10, when being operated, may be arranged with the axis in a vertical direction, and the fluid flow direction is along the axis from the fluid inlet 11 located at the bottom of the housing 10 to its fluid outlet 12 located at the top of the housing 10. Other shapes and arrangements of a housing 10 are, however conceivable and may be realized by a skilled person in accordance with the requirements of a specific case.

As a skilled person will appreciate, a treatment of biologically effective molecules with radiation - in the present invention: with UV radiation - requires the application of a minimum UV radiation dose in order to guarantee for an effective inactivation treatment of an allergenic molecule. Such an effective dose may be applied to the allergenic molecule, while flowing with the fluid flow to be treated, on its course along the length of the housing 11. This is why the housing is required to have a certain length and diameter. In preferred embodiments of the invention, the length of the housing 11, for example of the cylinder of the housing 11, is within a range of from 30 cm to 200 cm, more preferably within a range of from 80 cm to 150 cm, and the diameter may preferably be within a range of from 8 cm to 30 cm, more preferably within a range of from 10 cm to 20 cm. In a particularly advantageous example, the length of the housing is 100 cm, and its diameter is 16 cm, thereby allowing an effective treatment of a fluid flow containing biologically active molecules as, for example, allergenic molecules with inactivating UV radiation, without that such indications of the dimensions should be considered or interpreted to be a restriction of the invention.

The housing 10 of the apparatus 1 of the invention preferably extends in a vertical direction between the fluid inlet 11 and the fluid outlet 12, which are - for obvious reasons - preferably located at the two axial ends of the housing's cylinder 10. More preferably, the fluid inlet 11 of the housing 10 is located at its bottom end, and the fluid inlet 12 is located at its top end. The fluid inlet 11 and the fluid outlet 12 may be arranged to be in line with, or even symmetrical to, the cylinder axis of the housing 10, as is shown in Figure 1 as a particularly preferred embodiment, while also other arrangements of the fluid inlet 11 and/or fluid outlet 12, relative to the cylinder axis, are conceivable, for example in order to improve the turbulence of the fluid flow and or minimize the loss of pressure of the fluid flow, when being treated by the inactivating UV radiation. One example of a non-symmetrical arrangement of the fluid inlet 11 and/or the fluid outlet 12 may be the provision of a fluid inlet 11 and/or fluid outlet 12 in a certain angle relative to the cylinder axis. Due to this preferred arrangement of the housing 10, the fluid inlet 11 and the fluid outlet 12, an effective flow of the fluid to be treated by the UV radiation can be ensured, without that an excessive pressure loss of the fluid has to be expected.

Further, in accordance with the invention, the apparatus 1 of the invention comprises a means for establishing, and stimulating, a fluid flow from the fluid inlet 11 through the housing 10 to the fluid outlet 12. It has turned out beneficial for the apparatus 1 of the invention (see, for example, the document WO-A 2005/039,659) to employ a fan 20 as the means for establishing and stimulating a fluid flow from the fluid inlet 11 through the housing 10 to the fluid outlet 12. In preferred embodiments of the present invention, the fan may be positioned in the apparatus of the present invention suitable for a fluid treatment in such a manner that the fluid flow at least in a part of the housing 10, for example in the UV treatment chamber 30 (to be explained in detail below), is turbulent. Having turbulent fluid flows, for example fluid airflows, at least in a part of the housing 10, more preferably in the UV treatment chamber 30 (to be explained in detail below), has turned out to be beneficial and advantageous for the present apparatus and method for inactivating allergens in a fluid, for example in an airflow, by UV radiation treatment and, hence, is a preferred embodiment of the invention.

This means that the fan 20 may be positioned upstream relative to the UV treatment chamber 30, since the fluid flow stimulated by the fan 20 is always turbulent at the pressure side of the fan 20. In such a case, the fan 20 will always (i. e. independent upon the fluid flow rate) provide a turbulent fluid flow for passage through the UV treatment chamber.

At the side from where the fluid is drawn, the fluid flow may be laminar for relatively low fluid flow rates. However, it is noted that for high fluid flow rates, the flow is turbulent at the drawing side, too. Thus, in the apparatus 1 according to the present invention, the fan 20 may also be positioned downstream of the UV treatment chamber 30 when only using high fluid flow rates.

In order to provide a turbulent fluid flow, for example a turbulent air flow, through the housing 10 of the apparatus 1 of the present invention, more preferably through the UV treatment chamber 30, which turbulent fluid flow is preferred in view of the more effective UV radiation treatment of the allergenic components (and other components as, for example, one or more microorganism(s)) contained in the fluid as will be explained below in more detail, the inner wall of the fluid inlet 11 and/or of the housing 10 may be provided with means 14 increasing the fluid flow turbulence. As such means 14 for increasing the fluid flow turbulence generally result into a certain loss of pressure of the fluid flow, such means have to be carefully selected in accordance with the power (for example, without restricting the invention to this range, 20 to 200 W) the fan 20 may apply to the fluid flow for its passage through the housing 10 of the apparatus 1 of the present invention, as well as in accordance with the position the fan 20 has within the housing 10. By establishing the required balance between pressure/speed of the fluid low and the turbulence provided to the fluid flow, the effectiveness of the treatment of the fluid flow with UV radiation for achieving an inactivation of allergenic molecules, particularly proteinogenic allergen molecules, and also other components of the fluid forced to pass the apparatus of the invention can be improved considerably.

Suitable means 14 for increasing the fluid flow (e.g. airflow) turbulence in a housing passed by said fluid flow are well known to a skilled person and, hence, may be selected by a skilled person based on the usual experience in accordance with the requirements of the specific case, e.g. in accordance with the dimensions and/or arrangement of the housing 10, the power of the fan 20, the residence time of the fluid flow needed for an effective UV radiation treatment and other optional or essential parameters. One preferred example of means 14 for increasing the turbulence of the fluid flow are fins 14 which are arranged within the inner volume space of the housing 10 at a suitable place, e. g. at or close to the inner walls of the housing 10. Preferably, such fins 14 are located along the inner walls of the housing 10 upstream of the UV treatment chamber 30 (to be explained below) in order to generate the desired turbulence of the fluid flow before it enters into the UV treatment chamber. The size and exact location of the fins 14 may easily be determined for, and adapted to, the remaining parameters of the housing 10 by few orienting experiments carried out by a skilled person, in order to balance the pressure loss of the fluid flow and the turbulence provided to the fluid by the means 14 for increasing the turbulence. In accordance with the invention, the apparatus for treating a fluid comprises a UV treatment chamber 30 in said housing 10. Such a UV treatment chamber 30 may preferably be positioned downstream relative to said fluid inlet 11 in order to allow a maximum of incoming fluid to be treated by applying a UV radiation treatment. As may be seen from the sketch of Figure 1, the UV treatment chamber 30 takes a major part of the inner volume of the housing 10. The UV treatment chamber is characterized by being suitable to provide a maximum of the UV radiation generated therein to the fluid flow passing the UV treatment chamber, as well as to the components contained in said fluid flow, as for example the allergenic (e.g. proteinogenic allergen) molecules and optionally also other components.

The UV treatment chamber 30 comprises at least one UV radiation source 31 for exposing said fluid flow to UV radiation for inactivating allergenic molecules present in said fluid flow.

In accordance with the invention, there may be present one UV radiation source 31 in said UV treatment chamber 30 for emitting to said fluid flow UV radiation for inactivating allergenic molecules (and optionally also other components, for example microorganisms) present in said fluid flow, or there may be two or even more, for example three or four, UV radiation sources 31 for exposing said fluid flow to UV radiation for inactivating allergenic molecules (and optionally also other components) present in said fluid flow. Preferred examples of such UV radiation sources 31 are - without restriction - high pressure lamps or medium pressure lamps or low pressure lamps; UV radiation-emitting LEDs (light emitting diodes) may also be used advantageously. The invention may - generally - use all conceivable UV radiation-emitting light sources known in the art, particularly preferably all conceivable UV-C radiation-emitting light sources. In accordance with the invention, a use of different types of UV radiation-emitting light sources may also be envisaged; in one specific example, one UV radiation-emitting light sources selected from high pressure lamps, medium pressure lamps and low pressure lamps may be used together with one or more UV radiation-emitting LED(s), or several LEDs having the same or different UV radiation light spectrum or even a more or less narrow UV radiation wavelength range may be used, without restricting the invention to these exemplary embodiments.

Depending upon the type of UV radiation-emitting lamps used in the apparatus 1 of the present invention, there may be selected high pressure lamps, medium pressure lamps, low pressure lamps, UV radiation emitting LEDs or even one or several of the afore-mentioned types of lamps together, i. e. for a simultaneous or sequential emission/application of UV radiation onto fluid flows passing a volume of the housing 10 / of the UV treatment chamber 30 of the apparatus 1 of the invention. The type of lamps may be selected from a broad range of types known and available to a person skilled in this technical field. Actually, they may be selected in accordance with the requirements of a specific case, including (but not restricted to) the requirements resulting from the construction and/or size of the apparatus of the invention, from the type of connection to the source of electricity (one-socket lamps, two-socket lamps, plug-in connector lamps, etc.), from the UV radiation wavelength (or wavelength range(s)) required for an inactivation of allergens (and, optionally, other molecules) (see the detailed explanation below) and from other parameters.

In cases of using more than one UV radiation source 31, such two or more UV radiation sources 31 may emit UV light of the same wavelength range or UV light having different wavelength ranges. In one preferred embodiment, all UV light sources 31 emit light having the same (or at least closely related) wavelength range(s). Another preferred embodiment provides UV radiation sources (especially UV-C radiation-emitting sources), e. g. LEDs emitting one specific UV-(C) wavelength or a range of UV (-C) wavelengths which is very narrow and specified for a specific inactivation task. However, UV radiation sources emitting UV radiation having different wavelengths may be used advantageously, too. In particularly preferred embodiments, the wavelength range(s) of the UV radiation sources 31 may be specifically adapted, by a suitable selection in a single case, to radiation needed to inactivate specific allergens or specific "families" of related allergens. Namely, it was found in the frame of the present invention that emitted UV radiation of one specific wavelength (or at least of a specific wavelength range) is particularly suitable for inactivating specific allergens of protein origin. In particularly preferred embodiments of the invention, the range of the UV radiation emitted by the UV radiation sources 31 in the UV treatment chamber 30 of the apparatus 1 of the present invention is selected in accordance with the need for inactivating allergens of a specific origin or type, as will be explained below and in the experimental section by a suitable example.

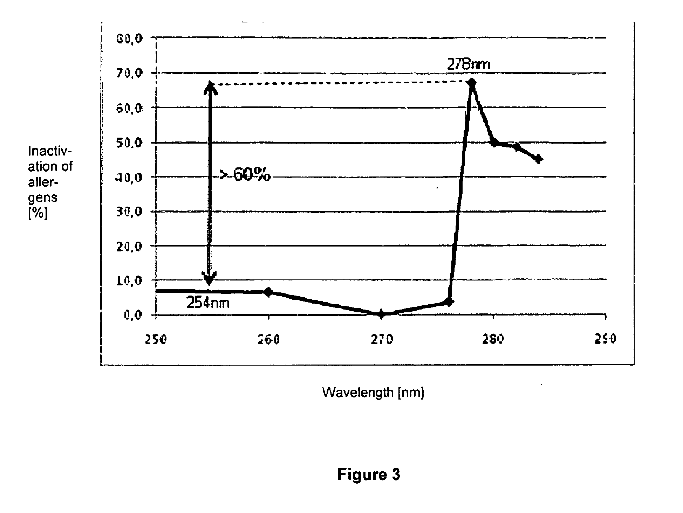

In preferred embodiments of the invention, the at least one UV radiation source 31 emits UV radiation in a wavelength range of at least 240 nm to 300 nm, preferably in a wavelength range of at least 250 nm to 285 nm, more preferably in a wavelength range of at least 270 nm to 280 nm. Particularly the latter UV wavelength range is suitable for the aim of the invention, i. e. the inactivation of allergens, specifically of proteinogenic allergens: As can be seen from Figure 3, UV light of a wavelength around 278 nm is absorbed by allergens in the course of an inactivation step by UV-C radiation treatment of a fluid flow, particularly of an air flow, containing such allergens, with the effect that particular allergens of protein origin are inactivated utmost effectively by irradiating a fluid flow containing such allergens, for example an air flow containing such allergens, with UV-C light having (or at least comprising to a measurable extent) UV-C light of a wavelength of 278 nm.

On the other hand, one preferred embodiment of the invention relates to an apparatus 1 of which two or more radiation sources emit UV radiation in the above range, and the two or more UV radiation sources may, for example emit UV-C radiation of 250 to 260 nm, more preferably 252 to 256 nm, specifically 254 nm (the latter being the wavelength having the peak intensity), in the case of at least one UV-C radiation source, and UV-C radiation of 270 to 285 nm, more preferably 275 to 280 nm, specifically 278 nm (the latter being the wavelength having peak intensity), in the case of at least one other UV-C radiation source.

Finally, the apparatus 1 for inactivating allergens in a fluid by UV radiation treatment in accordance with the present invention comprises control means 32a, 32b, 32c arranged in said fluid flow from the fluid inlet 11 through the housing 10 to the fluid outlet 12 so as to control parameters relevant for the allergen inactivation in said fluid. Non-restricting examples of such parameters relevant for the allergen inactivation and to be controlled by said control means 32a, 32b, 32c are the UV radiation dose applied, the flow speed of the fluid containing allergenic molecules, the temperature of the fluid treated, the humidity of the fluid treated, and other parameters. Suitable means for controlling these parameters, as for example (without restriction) UV radiation sensors for controlling the UV dose applied, manometers for controlling the pressure, e. g. by differential measurements, thermometers for controlling the temperature, hygrometers for controlling the humidity, etc., are well known to a skilled person and may be selected in accordance with the requirements and applied within the inner volume of the housing 10 so as to be in contact with the flowing fluid containing allergens, particularly allergens of protein origin ("proteinogenic allergens").

Particularly in cases of UV radiation sensors, specifically in the case of UV-C radiation sensors, it is preferred that the sensing wavelength of the sensor is adapted to the wavelength the corresponding UV radiation-emitting light source emits. This means that, in cases if two or more UV radiation emitting light sources, two or more UV radiation-sensing sensors are preferred. In an even more preferred embodiment, sensors capable of sensing UV radiation in a wavelength range of at least 240 nm to 300 nm, preferably in a wavelength range of at least 250 nm to 285 nm, more preferably in a wavelength range of at least 270 nm to 280 nm are preferred. In even more preferred embodiments of the invention (without restricting the invention to those sensors), two or more sensors capable of sensing UV radiation in the above range may be used, and the two or more UV radiation sensors may, for example be capable of sensing UV-C radiation of 250 to 260 nm, more preferably 252 to 256 nm, specifically 254 nm, in the case of at least one UV-C radiation sensor, and UV-C radiation of 270 to 285 nm, more preferably 275 to 280 nm, specifically 278 nm, in the case of at least one other UV-C radiation sensor.

Specifically, sensing the UV radiation emitted by the UV radiation emitting sources may be advantageous in view of the fact that the effect of the UV-C radiation emitted by the light sources can be controlled in the course of the time. UV-C radiation-emitting light sources having a certain UV-radiation emitting life time (of, for example, approximately 8,000 hrs) decrease in the UV-light emission intensity over the time. A decreasing intensity should be detected by suitable sensors in order not to decrease the allergen-inactivating capability of the UV radiation, specifically UV-C radiation of a certain wavelength found to effectively inactivate proteinogenic allergens, in the course of the time to a value below a desired lower limit. The decrease of intensity detected by a sensor of corresponding sensing capability may result, in the course of the process control, into an increase of UV radiation irradiation time.

A skilled person also knows, where in the fluid flow area, i. e. in the housing 10 between the fluid inlet 11 and the fluid outlet 12, such control means 32a, 32b, 32c may best be positioned, dependent upon a specific apparatus of the invention, and will provide, specifically affix, such control means 32a, 32b, 32c to the apparatus 1 of the invention, preferably to the inner wall of the housing 10 of the apparatus 1 of the invention, at a suitable place. For example, the UV radiation sensor(s) may be affixed to the inner wall of the apparatus 1 in the area which is covered by the UV radiation emitted by the UV radiation source. As one further example, the thermometer may be affixed ideally to the inner wall of the apparatus 1 at a place adjacent to the fluid inlet 11 in order to allow an adjustment of the temperature of the fluid flow fed to the housing 10 via the fluid inlet 11.

For other control means and/or their positioning within the fluid/air flow of the apparatus of the invention, reference may be made to the above-mentioned document WO 2005/039,659, the content of which is incorporated by reference into the present application: All control means disclosed in said document may also be used, each alone or two or more of them in combination, with the features of the apparatus of the present invention as described herein.

In preferred embodiments of the invention, the fluid treated in accordance with the invention is air. Allergens are transported by an air flow most often, either in a natural environment of a room (or in the free nature, from which the allergens enter into a room where humans live) or in an air flow artificially moved by air conditioning or artificial cleaning, humidifying, desodorising or tempering of indoor air. It is particularly preferred that such air provided to a room for living for humans be free of allergens completely.

In other preferred examples of the invention, the fluid treated by the apparatus of the invention is the air of a room exposed to allergens. Such an exposure to allergens may result from the fact that external allergens enter a room, for example via air conditioning or through an open window or door.

In cases of allergens of plant origin, hazelnut pollen, birch tree pollen or graminaceous pollen may be mentioned as examples. Such exposure to natural environmental allergens of protein origin may, however, also be the result of allergens already present in the room: House dust and mites may be one example for such allergens.

In another preferred example of the invention, the fluid treated is the air of a room not exposed to allergens, but contaminated by carriers of allergenic molecules. Such carriers (in the real sense of the word) may be pets or animals living in close connection to humans, like cats and dogs (which freely enter into the living environment of humans) or are used (like horses) for sports or for entertainment. While staying with these animals, humans contact these animals. The animals then either enter into the living space (like dogs or cats) or leave their "allergen print" on hands and clothing (like horses). Thus such allergens freely enter the humans' living environment. Thereby, air originally not exposed to allergens is "contaminated" and is often desired to be treated for allergen inactivation.

Hence, in preferred embodiments of the invention, allergens in the fluid or in the air are proteinogenic allergens. Preferably, the allergens in the fluid, specifically in the air, are selected from the group consisting of hazelnut pollen, birch tree pollen, graminaceous pollen, house dust and animal hair In even more preferred examples of the invention which, however, are not restricting, the allergens in the fluid, specifically in the air, are selected from the group consisting of equine hair, canine hair and feline hair bearing their animals' allergens.

The invention also relates to a method for inactivating allergens in a fluid by UV radiation treatment in an apparatus 1 as, for example, described above in detail, said apparatus 1 comprising
- a housing 10 comprising and extending between a fluid inlet 11 and a fluid outlet 12;
- a fan 20 for generating and stimulating a fluid flow from the fluid inlet 11 through the housing 10 to the fluid outlet 12;
- a UV treatment chamber 30 in said housing 10 preferably (but not necessarily) downstream relative to said fluid inlet 11, the UV treatment chamber 30 comprising at least one UV radiation source 31 for exposing said fluid flow to UV radiation for inactivating allergenic molecules present in said fluid flow; and
- control means arranged in said fluid flow from the fluid inlet 11 through the housing 10 to the fluid outlet 12 so as to control parameters relevant for the allergen inactivation in said fluid.

The method of the present invention comprises at least one of the following steps:
- guiding at least a part of said fluid flow through at least a part of the housing 10;
- directing said fluid flow towards, and at least partially into, said UV treatment chamber 30;
- exposing said fluid flow in said UV treatment chamber 30 to UV radiation for inactivating allergenic molecules present in said fluid flow; and
- controlling said exposure step, and optionally other steps in combination, with respect to at least one of the parameters relevant for the allergen inactivation in said fluid.

Preferably, the method for inactivating allergens in a fluid by UV radiation treatment in accordance with the present invention comprises at least the above four process steps of guiding at least a part of said fluid flow through at least a part of the housing 10; directing said fluid flow towards, and at least partially into, said UV treatment chamber 30; exposing said fluid flow in said UV treatment chamber 30 to UV radiation for inactivating allergenic molecules present in said fluid flow; and controlling said exposure step, and optionally other steps in combination, with respect to at least one of the parameters relevant for the allergen inactivation in said fluid in combination.

In another preferred embodiment of the invention, additional process steps may be included into the method of the invention.

A further preferred embodiment of the invention is directed to a method for inactivating allergens in a fluid by UV radiation treatment as described above and comprising at least the above four process steps, which method is conducted by operating the above apparatus 1 continuously or intermittently while controlling the exposure step, and optionally further process steps in combination, so as to be operated within a process conditions "window". Such a process conditions "window" is determined in advance, and/or is adapted continuously to requirements occurring, in the course of conducting the process, to optimum conditions for inactivating allergens, specifically allergens of protein origin, in a fluid flow, particularly in air, more particularly in air in the living environment of humans. Since the process itself does not allow a "real time" determination whether all, or at least the majority of, allergens in a fluid flow or airflow are inactivated by a passage of said fluid flow or airflow through the UV-C treatment chamber 30 of the apparatus 1 of the invention, optimum conditions have to be determined in advance:
In a fluid (e. g. an airflow) having passed the apparatus 1 of the present invention and having been treated therein by UV-C radiation with the aim or inactivating allergens, particularly those of protein origin, therein, the concentration or remaining proteinogenic allergens has to be determined in a secondary, off-line reaction, an example of which is shown in the experimental part of this application. If allergens have remained in a concentration considered not to be a satisfactory result, for example exceeding the allergen concentration required for the air in said room, the conditions of the method have to be adapted until to a point where the result satisfies. The process conditions allowing achieving a satisfactory result of the UV-C radiation treatment may then be varied in such a way that the result, i. e. the final concentration of one or several (e. g. proteinogenic) allergen(s) in said fluid flow or airflow, is still at least satisfactory, if not good or excellent and/or in accordance with the requirements. Those ranges of process conditions allowing achieving such a satisfactory / good / excellent result will then form the "window" of process conditions within which the method of inactivating allergens in accordance with the invention is to be conducted. Of course, the effect of the UV-C radiation treatment may be controlled by (off-line) tests of the concentration of remaining (i. e. not inactivated) allergenes.

Parameters to be determined for achieving optimum results may be (without being restricted to) starting allergen concentration, type of allergen(s) to be inactivated, wavelength of UV-C radiation used for the treatment, dose of UV-C radiation applied to the fluid flow or airflow, residence time of the fluid flow or airflow under UV-C radiation treatment, temperature and humidity of the fluid flow or airflow treated by UV-C radiation, to name only some of the parameters relevant for the effectiveness of the allergen inactivation treatment.

In another preferred embodiment of the invention, the process of the present invention is conducted (more preferably by using the apparatus 1 of the present invention as described in detail above), by subjecting a fluid flow, more preferably an air flow, within said UV treatment chamber 30 to UV radiation at a wavelength range of at least 240 nm to 300 nm, preferably at a wavelength range of at least 250 nm to 285 nm, more preferably at a wavelength range of at least 270 nm to 280 nm, for example at 254 nm and/or at 278 nm.

Even more preferred is an embodiment of the process of the present invention, wherein a fluid flow, more preferably an air flow, is subjected within said UV treatment chamber 30 to the UV radiation emitted by at least two UV radiation-emitting light sources 31, 31 capable of emitting UV-C radiation of 250 to 260 nm, more preferably 252 to 256 nm, specifically 254 nm (the latter being the wavelength having the peak intensity), in the case of at least one UV-C radiation source, and UV-C radiation of 270 to 285 nm, more preferably 275 to 280 nm, specifically 278 nm (the latter being the wavelength having peak intensity), in the case of at least one other UV-C radiation source.

In further preferred embodiments of the invention, an inactivation of proteinogenic allergens in accordance with the invention, as described above, particularly by using the apparatus 1 of the present invention also described in detail above, may be effected together with an inactivation of microorganisms in a fluid flow, for example in an air flow.

As exemplary microorganisms (considered not to be restricting to the invention), bacteria, viruses, funghi and yeasts may be mentioned as examples. An inactivation of bacteria and/or viruses by the combined process of the present invention is particularly preferred.

In the inactivation of microorganisms together with the inactivation of allergens according to the present invention, the above term "together with" may be understood, in the present claims and specification, as having the meaning that an inactivation of microorganisms and an inactivation of allergens, particularly (but not restricted to) proteinogenic allergens, as described in detail above, may occur simultaneously, i. e. by passing a volume flow of a fluid through a suitable apparatus, for example an apparatus as described in the present specification and, exemplarily, shown as a sketch in Figure 1, said volume flow containing one or more types of microorganisms mentioned above and one or more types of allergens also described in detail above.

Such a volume flow of a fluid, for example of air, may be treated by a UV-C treatment at one or more wavelength(s) suitable to inactivate the microorganisms contained in the fluid flow and at one or more wavelength(s) suitable to inactivate the allergen(s) contained in the fluid flow. Instead of passing the fluid flow through an apparatus while simultaneously applying a UV-C treatment at one or more suitable wavelengths for simultaneous microorganism and allergen UV-C radiation treatment, the treatment steps, optionally at different wavelengths, may occur sequentially (e. g. either first microorganism UV-C radiation treatment and then allergen UV-C radiation treatment or *vice versa).* Alternatively, other sequential treatments may be provided, with the aim of suitably effecting an inactivation of undesired microorganisms and allergens in the fluid flow all together, at least to an extent not leaving any detrimental concentration of active microorganism(s) and allergen(s) left finally.

Such a combined UV-C radiation inactivation treatment of one or more microorganisms and one or more allergens may be effected by combining the UV-C radiation treatment method of the present invention, effective for inactivating allergens, with any desired inactivation treatment for microorganisms known to a person skilled in the art.

One specific, albeit not restricting, example of a suitable combination of the method of the present invention with a microorganism inactivation method is a combination of the present method with the methods described in the patent documents EP-A 1 680 147, EP-A 1 949 002 and EP-A 2 155 270 of the present Applicants' predecessor of rights.

In a similar manner, either the apparatus of the present invention may be used, if desired: two or even more of them in cases of a desired sequential treatment, if this is considered necessary or desired. Alternatively, the apparatus of the present invention may be used in any combination with any of those apparatus described in any of the above patent applications. In combining these apparatus, either sequence of apparatus desired may be made use of. A person skilled in the art may use these apparatus or methods for achieving the desired aim of inactivation of one or more microorganism(s) together with the inactivation of one or more allergen(s) in accordance with the present invention.

In particularly preferred embodiments of the invention, a UV radiation treatment, even more preferably: a UV-C radiation treatment, may be effected. This UV radiation treatment is suitably effected by passing a fluid flow, more preferably an air flow, containing one allergen or more than one allergen, for example two or three allergens, in preferred embodiments one or more proteinogenic allergen(s), and one microorganism or more than one microorganism, for example two or three microorganisms, to UV radiation, more preferably to UV-C radiation.

In even more preferred embodiments of the invention, the proteinogenic allergens to be inactivated by UV radiation are selected from the group consisting of hazelnut pollen, birch tree pollen, graminaceous pollen, house dust and animal hair. In even more preferred embodiments, the allergens in the fluid flow or airflow are selected from the group consisting of equine, canine and feline hair. It is even more preferred that the proteinogenic allergens are selected from the allergens Cor A1, Can f1, Phl p5, and Der p1.

On the other hand, the microorganisms to be inactivated by UV radiation together with allergens are selected from the group consisting of bacteria, viruses, funghi and yeasts. An inactivation of bacteria and/or viruses by the combined process of the present invention, i. e. by the combined UV treatment of a fluid flow, specifically by an airflow, for the inactivation of allergen(s) together with microorganisms is particularly preferred.

In another preferred embodiment of the invention, the process of the present invention for inactivating one or more allergen(s) together with one or more microorganism(s) is conducted (more preferably by using the apparatus 1 of the present invention as described in detail above), by subjecting a fluid flow, more preferably an air flow, within said UV treatment chamber 30 to UV radiation at a wavelength range of at least 240 nm to 300 nm, preferably at a wavelength range of at least 250 nm to 285 nm, more preferably at a wavelength range of at least 270 nm to 280 nm, for example at 254 nm and/or at 278 nm.

Even more preferred is an embodiment of the process of the present invention for inactivating one or more allergen(s) together with one or more microorganism(s), wherein a fluid flow, more preferably an air flow, is subjected within said UV treatment chamber 30 to the UV radiation emitted by at least two UV radiation-emitting light sources 31, 31 capable of emitting UV-C radiation of 250 to 260 nm, more preferably 252 to 256 nm, specifically 254 nm (the latter being the wavelength having the peak intensity), in the case of at least one UV-C radiation source, and UV-C radiation of 270 to 285 nm, more preferably 275 to 280 nm, specifically 278 nm (the latter being the wavelength having peak intensity), in the case of at least one other UV-C radiation source.

The present invention will be further explained by the following experimental example. This example, however, is directed to a preferred embodiment of the invention and should not be construed to limit the scope of the invention to the case exemplified in the following Examples.

### Examples

The effectiveness of a UV-C radiation treatment for an inactivation of allergens was practically exemplified as follows:
There was established an apparatus for the inactivation of allergens, particularly of allergens of protein origin, said device allowing inactivating proteinogenic allergens by UV-C radiation and allowing determining the optimum wavelength optimum wavelength range of UV-C radiation to be applied onto a fluid flow, especially onto an airflow, containing proteinogenic allergens of different types.
Allergens, in cuvettes made of quartz glass, of the following list were irradiated by photometer UV-C radiation of varying wavelengths. Subsequently, the antigens' specific antibody bonding was examined in ELISA (enzyme-linked immunosorbent assay) tests.

### List of proteinogenic allergens

| **Origin (gen.)** | **Origin (specific)** | **Allergen Denomination** |
|---|---|---|
| Plant | Hazelnut | Cor a1 |
| Plant | ("Wiesenlischgras" German) | Phl p5 |
| Animal | Dog (dog's hair) | Can f1 |
| Animal | House dust mite | Der p1 |

The tests were conducted as follows:
(A) Air "contaminated" by a predetermined amount of an allergen (the simultaneous measurement of more than one allergen of the above list was also possible in an analogous manner) was passed through the apparatus of the present invention. The allergens tested separately were those shown in the above list. The allergens tested were micronized from an allergen solution by a Pari Micronizer into the airflow of the apparatus 1 according to the invention.

The allergen solution to be micronized was obtained by separating allergen(s) from an airflow on a Gelatine filter, transferring the allergen-containing Gelatine filter into a PBS buffer solution (pH: 7.2), incubating at 38 °C for 10 min, thereby dissolving the Gelatine filter and using the solution obtained (having a suitable allergen concentration determined in advance by three calibrating experiments) in an amount of 1 ml or 2 ml for the subsequent test runs.

In a control run, the apparatus was operated first without UV-C treatment under the following conditions:

| | |
|---|---|
| Temperature: | 15 to 40 °C; |
| Humidity: | 70 ° rel. humidity; |
| Air Flow: | 1.5 m/s; |
| Length / diameter UV-C treatment chamber: | 100 cm / 16 cm. |

A defined partial volume of the airflow having passed the UV-C treatment chamber (without UV-C radiation emitting light sources being switched on) was separated and allowed to pass a Gelatine filter. Thereby, the allergen contained in the air flow could be collected on the filter.

The Gelatine filter was transferred into 30 ml PBS buffer (pH: 7.2) and was incubated at 38 °C for approximately 10 min. The filter was dissolved completely, and the respective allergen was released into the buffer.

If the resulting incubated allergen solution was subjected to an allergen-specific ELISA with an antigen-specific antibody, approximately 66 ± 2 % of the antigen used in the air flow having entered and passed the apparatus was found to be bound to the specific antibody. This result was considered to be the control result.

In parallel control runs, the amount of allergen micronized into the airflow and the air speed passing the apparatus were varied; the results obtained were more or less in the same range of the above control measurement.

Further control runs were made in accordance with the same schedule described above; the airflow was, however, treated by a heat treatment. Practically, the heat treatment was effected by switching the UV-C radiation source on, but keeping said UV-C radiation source wrapped by a piece of aluminium foil allowing the heat (generated by the UV-C radiation source) to be radiated onto the airflow passing through the treatment chamber 30 without that the UV-C radiation was allowed to be irradiated onto the airflow containing the proteinogenic allergens. Thereby, the heat treatment without UV-C irradiation was effected at a elevated temperature.

In the runs for determining the effectiveness of the allergen inactivation by UV radiation, the experiments were conducted in the same way as described above, with the exception that the UV-C radiation source was switched on. The UV-C radiation source applied approximately the same heat onto the airflow as in the previous heat application test run. The irradiated UV-C radiation had a wavelength in the range of from 250 nm to 280 nm. Specifically, wavelengths of 254 nm and 278 nm were tested for their effect on an allergen inactivation. Also in these cases, a partial volume of the air having passed the UV treatment chamber was collected, allowed to pass a Gelatine filter (on which the allergens were separated); and the filter was transferred into the PBS buffer solution, dissolved and the allergens were released. Allergens in the solution were bound in ELISA tests to specific antibodies, and the amount of bound allergen was determined.

In all control and test runs, there was only one singular passage of the allergen-containing airflow through the apparatus of the present invention.

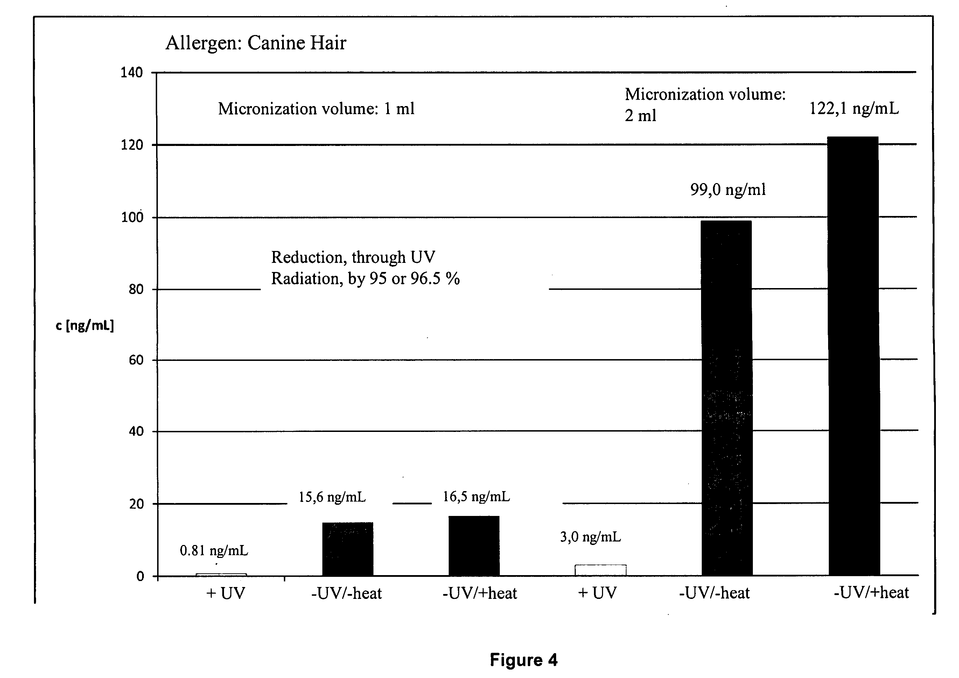

The results of:
- Control run without heat ("- heat") and without UV-C radiation ("- UV");
- Control run with heat ("+ heat") and without UV-C radiation ("- UV"); and
- Test run with heat and with UV-C radiation ("+ UV") of 254 nm wavelength at a UV-C radiation intensity of 45 mW/cm²
for the two allergens Can f1 (canine hair allergen) and Cor a1 (hazelnut allergen) are shown in the enclosed Figures 4 (Can f1) and 5 (Cor 1a) for two different amounts of allergen solution (1 ml; 2ml) micronized into the airflow.

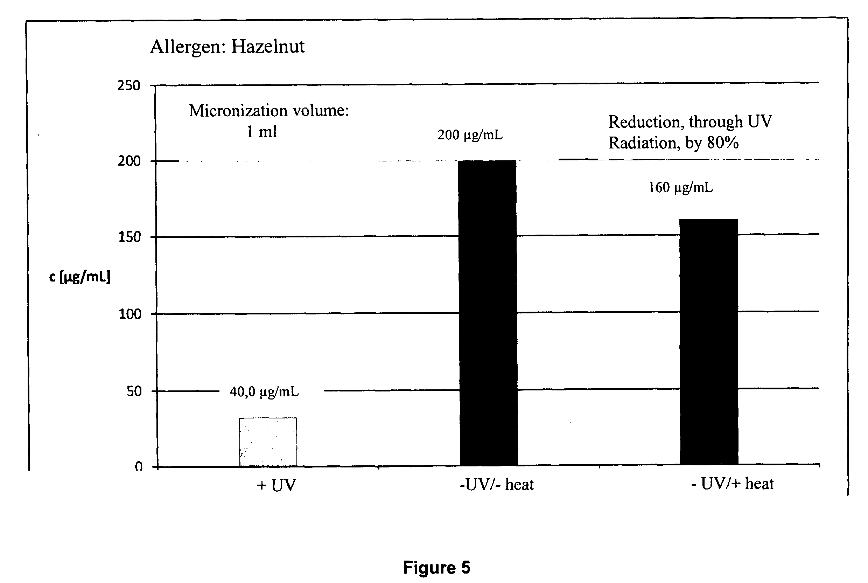

The following results may be derived from Figures 4 and 5:
(a) Air having passed the apparatus of the present invention without UV-C treatment and without heat application contained the full amount of allergen (considering the "loss" by the separation on the Gelatine filter and subsequent binding to antibodies in ELISA) entered into the control run (see the respective middle columns in the triples of columns of the experiments represented by Figures 4 and 5.
(b) Air having passed the apparatus of the present invention without UV-C treatment and with heat application contained a slightly increased (canine hair allergen) / reduced (hazelnut allergen) amount of allergen (also considering the "loss" by the separation on the Gelatine filter and subsequent binding to antibodies in ELISA) entered into the control run (see the respective right hand columns in the triples of columns of the experiments represented by Figures 4 and 5).
(c) Air having passed the apparatus of the present invention with UV-C treatment and with heat application contained substantially lower amount of allergen (considering the "loss" by the separation on the Gelatine filter and subsequent binding to antibodies in ELISA) entered into this test run (see the respective left hand columns in the triples of columns of the experiments represented by Figures 4 and 5).

In the case of the canine allergen Can f1 (Figure 4), the reduction of the amount of "active" allergen (being capable, even after the UV-C radiation treatment, of binding to the specific antibody) is from 15.6 ng/ml to 0.9 ng/ml (corresponding to a reduction to 5 % or a reduction by 95 %) for the lower allergen solution micronization concentration and from 99.0 ng/ml to 3,0 ng/ml (corresponding to a reduction to 3.5 % or a reduction by 96.5 %) for the higher allergen solution micronization concentration.

In the case of the hazelnut allergen Cor a1, the reduction of the amount of "active" allergen (being capable, even after the UV-C radiation treatment, of binding to the specific antibody) is from 200 µg/ml to 40.0 µg/ml (corresponding to a reduction to 20 % or a reduction by 80 %); only the lower allergen solution micronization concentration of 1 ml was tested.

Similar results were achieved with the Der p1 and Phl p5 allergens listed above.

(B) A further test under the same general conditions was conducted with the hazelnut allergen Cor a1. The air containing the allergen was treated with UV-C radiation of the wavelengths 254 nm and 278 nm at identical intensities. The percentage of allergen inactivation, surprisingly, was more than 60 % higher at 278 nm, compared to 254 nm. This is shown in Figure 3.

It is concluded from this result that the inactivation of allergens (exemplified by Cor a1: hazelnut allergen) with UV-C radiation at 278 nm is considerably more effective than with UV-C radiation at 254 nm. Moreover, it can be concluded from the above result that the inactivation of allergens, at least of those of protein origin, may be effected in a highly effective manner at a UV-C radiation wavelength of approx. 278 nm.

With the above apparatus and method, it is surprisingly possible to inactivate allergens, particularly proteinogenic allergens and more specifically proteinogenic allergens selected from the group consisting of hazelnut pollen, birch tree pollen, graminaceous pollen, house dust and animal hair, and even more specifically the proteinogenic allergens in a fluid selected from the group consisting of equine, canine and feline hair, from fluids and specifically from air of human living environment. Hence, such proteinogenic allergens are no longer present in the indoor air and do no longer harm humans living in such rooms previously contaminated with the allergens.

Even more surprisingly, an effective inactivation of allergens, preferably proteinogenic allergens, together with microorganisms by UV-C radiation treatment could be achieved.

The invention is not restricted to the preferred embodiments described above for exemplary purposes and given for allowing a better understanding of the invention.

## Claims

1. An apparatus (1) for inactivating allergens in a fluid by UV radiation treatment, said apparatus (1) comprising
- a housing (10) comprising and extending between a fluid inlet (11) and a fluid outlet (12);
- a fan (20) for generating and stimulating a fluid flow from the fluid inlet (11) through the housing (10) to the fluid outlet (12);
- a UV treatment chamber (30) in said housing (10) downstream relative to said fluid inlet (11), the UV treatment chamber (30) comprising at least one UV radiation source (31) for exposing said fluid flow to UV radiation for inactivating allergenic molecules present in said fluid flow; and
- control means (32) arranged in said fluid flow from the fluid inlet (11) through the housing (10) to the fluid outlet (12) so as to control parameters relevant for the allergen inactivation in said fluid.

2. The apparatus according to claim 1, wherein the fluid treated is air, preferably wherein the fluid treated is the air of a room exposed to allergens, more preferably wherein the fluid treated is the air of a room not exposed to allergens, but contaminated by carriers of allergenic molecules.

3. The apparatus according to claim 1 or claim 2, wherein the allergens in the fluid are proteinogenic allergens, preferably wherein the allergens in the fluid are selected from the group consisting of hazelnut pollen, birch tree pollen, graminaceous pollen, house dust and animal hair, more preferably wherein the allergens in the fluid are selected from the group consisting of equine, canine and feline hair, even more preferred wherein the proteinogenic allergens are selected from the allergens Cor A1, Can f1, Phl p5, and Der p1.

4. The apparatus according to any one of the claims 1 to 3, wherein the at least one UV radiation source (31) emits UV radiation in a wavelength range of at least 240 nm to 300 nm, preferably in a wavelength range of at least 250 nm to 285 nm, more preferably in a wavelength range of at least 270 nm to 280 nm, for example at 254 nm and/or at 278 nm.

5. The apparatus according to claim 4, wherein at least two UV radiation sources (31, 31) emit UV-C radiation of 250 to 260 nm, more preferably 252 to 256 nm, specifically 254 nm (the latter being the wavelength having the peak intensity), in the case of at least one UV-C radiation source, and UV-C radiation of 270 to 285 nm, more preferably 275 to 280 nm, specifically 278 nm (the latter being the wavelength having peak intensity), in the case of at least one other UV-C radiation source.

6. The apparatus according to any one of the claims 1 to 5, wherein the apparatus has at least one of the following features alone or in a combined manner in combination with any of the features of the claims 1 to 5:
- the housing (10) has a cylindrical shape; preferably the housing (10) has a cylindrical shape with a vertically arranged axis; even more preferably the housing (10) has a cylindrical shape with the fluid inlet (11) being located at the bottom of the housing (10) and the fluid outlet (12) being located at the top of the housing (10);
- the housing (10) has a length in the range of from 30 to 200 cm, preferably in the range of from 80 to 150 cm, and/or the housing (10) has a diameter in the range of from 8 to 30 cm, preferably in the range of from 10 to 20 cm; more preferably wherein the housing (10) has a length of about 100 cm and/or a diameter of about 16 cm,
- the fluid inlet (11) and/or the fluid outlet (12) of the housing (10) are arranged in line with the axis of the cylinder of the housing (10), preferably are arranged symmetrically with respect to the housing's cylinder axis; or the fluid inlet (11) and/or the fluid outlet (12) of the housing (10) are arranged off-line with respect to the axis of the cylinder of the housing (10), preferably are arranged non-symmetrically with respect to the housing's cylinder axis; more preferably wherein the fluid inlet (11) and/or the fluid outlet (12) are arranged at a certain angle relative to the cylinder axis;
- the housing (10) comprises the fan (20) at a position within the housing suitable for generating a turbulent fluid flow from the fluid inlet (11) to the fluid outlet (12), preferably the housing (10) comprises the fan (20) upstream of the UV treatment chamber (30);
- the housing (10) comprises means (14) for generating a turbulent fluid flow within the housing (10), preferably through the UV treatment chamber (30), more preferably in the form of fins (14) arranged at the inner housing walls;
- the UV treatment chamber (30) accommodates at least one UV radiation source (31), said one or more radiation source(s) (31) being selected from high-pressure UV radiation-emitting lamps, medium-pressure UV radiation-emitting lamps, low-pressure UV radiation-emitting lamps, and UV radiation-emitting LED's;
- the UV treatment chamber (30) comprises - preferably on the inner wall of the housing (10) in the area of the UV treatment chamber - a UV light reflecting layer;
- the control means (32) comprise UV radiation sensors, manometers, thermometers, hygrometers, flow speed meters.

7. A method for inactivating allergens in a fluid by UV radiation treatment in an apparatus comprising
- a housing (10) comprising and extending between a fluid inlet (11) and a fluid outlet (12);
- a fan (20) for generating and stimulating a fluid flow from the fluid inlet (11) through the housing (10) to the fluid outlet (12);
- a UV treatment chamber (30) in said housing (10) downstream relative to said fluid inlet (11), the UV treatment chamber (30) comprising at least one UV radiation source (31) for exposing said fluid flow to UV radiation for inactivating allergenic molecules present in said fluid flow; and
- control means arranged in said fluid flow from the fluid inlet (11) through the housing (10) to the fluid outlet (12) so as to control parameters relevant for the allergen inactivation in said fluid; and
- optionally one or more than one of the features of claim 6;
- said method comprising at least one of the following steps:
- guiding at least a part of said fluid flow through at least a part of the housing (10);
- directing said fluid flow towards, and at least partially into, said UV treatment chamber (30);
- exposing said fluid flow in said UV treatment chamber (30) to UV radiation for inactivating allergenic molecules present in said fluid flow; and
- controlling said exposure step, and optionally other steps in combination, with respect to at least one of the parameters relevant for the allergen inactivation in said fluid.

8. The method in accordance with claim 7, which method is conducted by operating the apparatus as claimed in any of the claims 1 to 6 continuously or intermittently, while controlling the UV radiation exposure step, and optionally further method steps, so as to be operated within a predetermined process conditions window.

9. The method in accordance with any one of the claims 7 or 8, which method is conducted by subjecting a fluid flow, preferably an airflow, within said UV treatment chamber (30) to UV radiation at a wavelength range of at least 240 nm to 300 nm, preferably at a wavelength range of at least 250 nm to 285 nm, more preferably at a wavelength range of at least 270 nm to 280 nm, for example at 254 nm and/or at 278 nm.

10. The method in accordance with claim 9, which method is conducted by subjecting a fluid flow, preferably an air flow, within said UV treatment chamber (30) to the UV radiation of two or more UV radiation sources (31, 31) capable of emitting UV-C radiation of 250 to 260 nm, more preferably 252 to 256 nm, specifically 254 nm (the latter being the wavelength having the peak intensity), in the case of at least one UV-C radiation source, and UV-C radiation of 270 to 285 nm, more preferably 275 to 280 nm, specifically 278 nm (the latter being the wavelength having peak intensity), in the case of at least one other UV-C radiation source.

11. The method in accordance with any one of the claims 7 to 10, wherein the inactivation of allergens is conducted together with the inactivation of microorganisms, preferably either simultaneously or sequentially.

12. The method of any of the claims 7 to 11, wherein the inactivation of allergens is conducted together with the inactivation of bacteria and/or viruses, either simultaneously or sequentially.

13. The method of any of the claims 7 to 12, wherein the inactivation of allergens is conducted together with the inactivation of microorganisms by subjecting a fluid flow, preferably an airflow, within said UV treatment chamber (30) to UV radiation at a wavelength range of at least 240 nm to 300 nm, preferably at a wavelength range of at least 250 nm to 285 nm, more preferably at a wavelength range of at least 270 nm to 280 nm, for example at 254 nm and/or at 278 nm.

14. The method of claim 13, wherein the inactivation of allergens is conducted together with the inactivation of microorganisms by subjecting a fluid flow, preferably an airflow, within said UV treatment chamber (30) to UV radiation of two or more UV radiation sources (31, 31) capable of emitting UV-C radiation of 250 to 260 nm, more preferably 252 to 256 nm, specifically 254 nm (the latter being the wavelength having the peak intensity), in the case of at least one UV-C radiation source, and UV-C radiation of 270 to 285 nm, more preferably 275 to 280 nm, specifically 278 nm (the latter being the wavelength having peak intensity), in the case of at least one other UV-C radiation source.
